# EUROPÄISCHE PATENTSCHRIFT

(11) **EP 2 067 498 B1**
(45) Veröffentlichungstag und Bekanntmachung des Hinweises auf die Patenterteilung: **01.02.2012**
(21) Anmeldenummer: 07122132.9
(22) Anmeldetag: 03.12.2007
(51) Int. Cl.: A61M 25/01

(54) **Katheterstilett mit Katheter-Aufnahmelumen**
Catheter with catheter receptacle lumen
Stylet de cathéter doté d'une lumière de saisie de cathéter

(43) Veröffentlichungstag der Anmeldung: 10.06.2009
(73) Patentinhaber: BrainLAB AG, 85622 Feldkirchen (DE)
(72) Erfinder: Mittermeyer, Stephan, 84036, Landshut (DE); Pedain, Christoph, 81667, München (DE)
(74) Vertreter: Schwabe - Sandmair - Marx

(56) Entgegenhaltungen:
- WO-A-2004/062718
- US-A- 5 098 411
- US-A- 5 665 052
- US-A1- 2004 138 562

## Beschreibung

Die Erfindung betrifft das technische Gebiet der Stilett-Versteifung eines Katheters während des Katheter-Setzvorganges. Insbesondere betrifft sie ein Katheterstilett, und speziell ein Stilett für einen Katheter, der im Rahmen einer CED-Behandlung (Convection Enhanced Delivery) verwendet wird.

Eine CED-Behandlung, also eine Convection-Enhanced-Delivery-Behandlung ist eine neurochirurgische Anwendung, bei der ein Katheter in festes Gehimgewebe eingebracht wird, um dort über längere Zeit und in sehr kleinen Mengen einen Wirkstoff bzw. ein Medikament abzugeben. Damit ein solcher Katheter entlang einer geplanten Trajektorie gesetzt werden kann, wird ein Stilett benötigt, welches die notwendige Stabilität für den Katheter beim Setzen bereitstellt, um alle Oberflächen im Gewebe durchdringen zu können. Nach dem Setzvorgang wird das Stilett entfernt, und der im Gewebe verbleibende Katheter ist dann gemäß seinem Material weich bzw. flexibel, sodass Traumata verhindert werden.

Aus der US 2004/0215143 A1 ist ein hohles Stilett für ein Infusionskathetersystem bekannt, wobei das Hohlstilett mit einen. Medikament befüllt ist, während der Katheter mit dem Stilett zusammen gesetzt wird. Bei der Entfernung des Stiletts, welches zum Setzen in der flüssigkeitsführenden Katheterleitung untergebracht war, soll das im hohlen Stilett eingesetzte Medikament in der Katheterleitung verbleiben, und dadurch wird der Aufbau von Luftblasen in der Katheterleitung nach der Entfernung des Stiletts minimiert.

Ein Rückfluss-Katheter mit einem hohlen Stilett ist aus der US 5,098,411, bekannt, und die US 4,046,144 beschreibt eine Katheter-Setzanordnung mit einer starrren Nadel und einem speziellen Anschlusssystem.

Das Dokument US 5,665,052 offenbart ein Laryngoskop mit einem zusätzlichen Katheter. Der Katheter ist von einem Stilett umgeben, das wiederum in einer endotrachealen Röhre angeordnet ist.

Das Dokument US 3,472,232 offenbart einen flexiblen Katheter, der in eine steifwandige Kanüle eingebracht werden kann. Dazu weist die Kanüle einen Längsschlitz auf.

Das Dokument US 5,318,542 offenbart eine Kanüle zur Aufnahme einer Röhre. Die Kanüle weist Angriffspunkte und Sollbruchstellen auf, um die Kanüle zu teilen und von der Röhre abzunehmen.

Das Dokument US 4,921,479 offenbart eine Hülle zur Aufnahme eines Katheters. Die Hülle besteht aus einem gerollten Kunststoffelement mit Formgedächtnis. Zur Entfernung des Katheters aus der Hülle kann diese radial aufgeweitet werden, so dass sich eine Längsöffnung ergibt.

Katheter-Setzsysteme mit Stiletten, die nicht hohl sind, haben in noch stärkerem Maße das Problem, dass beim Entfernen des Stiletts Luft in der flüssigkeitsführenden Katheterleitung verbleibt. Solche Luftblasen sind dabei speziell auf dem Gebiet der CED-Anwendungen äußerst problematisch. Die Luft dringt zuerst in das Verabreichungsgebiet ein und fordert dort Raum. Dies führt zu einem verlängerten Rückflussgebiet und einer nicht vorhersagbaren Medikamentenverteilung im Gewebe, was wiederum das Behandlungsergebnis in Frage stellen kann. Luft dringt hauptsächlich aus zwei Gründen in den Katheter vor. Ein Grund ist das Entfernen des Stiletts, das in der flüssigkeitsführenden Katheterieitung angeordnet war, wie oben schon beschrieben wurde. Ein weiterer Grund liegt in dem Verbindungsprozess mit der Medikamenten-Versorgungseinheit. Während dieses Verbindungsprozesses wird ebenfalls Luft in die infusionsleitung eingebracht, weil die Luft in dem aufnehmenden Teil des Luer-Adapters durch den eindringenden Teil des Luer. Adapters in die Leitung geschoben wird.

Es ist die Aufgabe der vorliegenden Erfindung, mindestens einige der oben beschriebenen Nachteile zu überwinden. Insbesondere soll eine Katheterstilett-Ausführung bereitgestellt werden, welche die Handhabung und das Setzen des Katheters vereinfacht' und Behandlungsergebnisse, insbesondere bei CED-Anwendungen, verbessert.

Diese Aufgabe wird durch ein Katheterstilet-Katheter-System gemäß dem Anspruch 1 und ein Verfahren gemäß dem Anspruch 15 gelöst Die Unteransprüche definieren bevorzugte Ausführungsformen der Erfindung.

Das Katheterstilett gemäß der vorliegenden Erfindung ist so ausgestaltet, dass es ein Aufnahmelumen für zumindest eine flüssigkeitsführende Leitung des Katheters aufweist. Mit anderen Worten ist das erfindungsgemäße Stilett eines, welches die flüssigkeitsführende Leitung des Katheters in dem Aufnahmelumen aufnehmen kann. Diese Lösung ist also genau entgegengesetzt zum Stand der Technik, wo das Stilett immer in der flüssigkeitsführenden Leitung des Katheters untergebracht wird, während der Katheter gesetzt wird.

Die erfindungsgemäße Lösung hat vielfältige Vorteile. Einer der wichtigsten betrifft natürlich die Tatsache, dass ein Katheter, der von Außen durch ein erfindungsgemäßes Stilett gestützt wird, mit einem Medikament vorbefüllt, d. h. "geprimed" werden kann. Das außen befindliche Stilett kann dann entfernt werden. ohne dass sich an der Vorbefüllung des Katheters etwas ändert, und insbesondere auch ohne dass die flüssigkeitsführende Leitung nochmals geöffnet werden muss, um das Stilett herauszunehmen. In dieser Weise wird ein Eindringen von Luft in den Katheter positiv vermieden. Außerdem kann der Katheter schon vorab mit seiner Versorgungseinrichtung (Spritze, Pumpe für das Medikament) verbunden werden, und diese Verbindung muss nicht mehr geöffnet werden, um das Stilett zu entfernen. Ein Eindringen von Luft aufgrund von Anschlussvorgängen kann also ebenfalls vermieden werden.

Die Erfindung nutzt ferner den Umstand, dass ein hohles Stilett, also ein Stilett mit einem Aufnahmelumen, im Wesentlichen cieselbe Steifigkeit aufweisen kann, wie Vollmaterial-Stilette, insbesondere wenn es - wie bei der vorliegenden Erfindung - außen um eine Leitung herum gelegt wird und deshalb schon einen etwas größeren Durchmesser aufweist. Aus diesem Grund kann die Versteifungsfunktion des Stiletts mindestens ebenso gut, wenn nicht besser als beim Stand der Technik erfüllt werden, und der Setzvorgang kann ohne Weiteres vorgegebenen Trajektorien folgen.

Bei der Erfindung ist am Stilett ein Zugang für das Aufnahmelumen vorhanden. Das Stilett weist einen im Wesentlichen zylinderförmigen oder rohrförmigen, länglichen Körper auf, und das Aufnahmelumen ist in diesem Fall der Innenraum des Körpers. Es besteht die Möglichkeit, den Zugang derart zu gestalten oder zu schaffen, dass das Stilett seitlich bzw. im Wesentlichen quer zum Verlauf der Leitung um diese herum angebracht oder von dieser abgezogen werden kann.

Über den genannten Zugang kommt also die flüssigkeitsführende Leitung des Katheters in das Stilett hinein. Ein solcher Zugang kann ein Längsschlitz im Stilettkörper sein, eine längs bzw. axial am Stilettkörper verlaufende Schwächungsstelle, insbesondere eine Perforation des Stilettmaterials, oder der Zugang kann zwei überlappende Längskanten am Körper des Stiletts umfassen. Es ist möglich, den Stiletfkörper als eine gerollte Folie auszubilden, und am Körper des Stiletts kann eine Hebeleinrichtung angeordnet werden, mit welcher das Stilett gegriffen oder im Durchmesser verändert werden kann, insbesondere zum Öffnen oder Schließen des, Zugangs, der durch zwei überlappende oder aneinander stoßende Stilettkörper-Längskanten gebildet wird. Ein gerolltes Folien-Stilett hat einerseits eine ausreichende Steifigkeit auf, um den Katheter gut setzen zu können, andererseits ist es flexibel genug, um aufgerollt zu werden, so dass man es von dem Katheter entfernen kann.

Das Stilett kann mehrere Material- oder Folienschichten umfassen, insbesondere eine unterschiedliche Anzahl von Schichten entlang der Stilettlänge. Dadurch kann auch ein konisch geformtes Stilett entstehen, ebenso wie durch mindestens einen im Winkel zusammengerollten Materialstreifen, insbesondere Folienstreifen. Ein solches, etwas konisch geformtes Stilett ist einfach entfernbar, es kann aber auch unterschiedliche Steifigkeiten entlang der Stilett-Länge bereitstellen, wenn dies notwendig ist.

Bei einer weiteren Ausführungsvariante der Erfindung ist das Stilett teleskopartig aufgebaut bzw. ein Stilettkörper mit einem etwas geringeren Durchmesser ruht in einem anderen Stilettkörper. Dabei können beide Stilettkörper einen offenen Zugangs-Längsspalt aufweisen, und diese Spalte müssen übereinander gelegt werden, wenn das Stilett seitlich vom Katheter zu entfernen ist. Solche ineinander liegenden oder Teleskop-Stilette können eine Längenverstellung aber auch eine Steifigkeitserhöhung ermöglichen.

Ein Katheterstilett gemäß der vorliegenden Erfindung kann eines oder mehrere der folgenden Materialien aufweisen oder aus einem Material hergestellt sein, das eine oder mehrere der folgenden Materialeigenschaften aufweist:
- ein Metall bzw. eine Metallfolie;
- ein Kunststoff bzw. eine Kunststoftfolie, insbesondere ein Polymer, speziell PE, PP oder PEEK;
- ein Kompositmaterial bzw. eine Kompositmaterialfolie;
- ein Memory-Metall oder eine Memory-Legierung;
- ein CT-kompatibles bzw. auf CT-Bildern sichtbares Material;
- ein MR-kompatibles bzw. auf MR-Bildern sichtbares Material;
- ein biokompatibles Material.

Die Erfindung betrifft ein Katheterstilett-Katheter-System mit einem Stilett, wie es oben in verschiedenen Ausführungsformen erörtert wurde, und mit einem Katheter, der eine flüssigkeitsführende Leitung aufweist.

Gemäß dieses erfindungsgemäßen Systems hat der Katheter eine innere flüssigkeitsführende Leitung und einen äußeren Hüllkatheterteil, und das Stilett ist zum Einsetzen zwischen die Leitung und den Hüllkatheterteil vorgesehen, so dass es die Leitung umgreift und vom Hüllkatheterteil umgriffen wird bzw. werden kann.

Bei einem System gemäß der vorliegenden Erfindung kann an der flüssigkeitsführenden Leitung eine Abzieh- oder Öffnungsvorrichtung angeordnet sein, um das Abziehen bzw. Öffnen des Stiletts zu erleichtern. Speziell ist es möglich, diese Vorrichtung als Kegel- oder Trichterelement auszugestalten.

Die Erfindung umfasst ferner ein Verfahren zum Versteifen eines Katheters vor dem Setzvorgang bei dem ein Stilett, das ein Aufnahmelumen aufweist, um zumindest eine flüssigkeitsführende Leitung des Katheters angeordnet wird. Dabei wird ein Stilett verwendet, wie es oben in verschiedenen Ausführungsvarianten beschrieben worden ist.

Die Erfindung wird im Weiteren anhand von Ausführungsformen und mit Hilfe der beiliegenden Zeichnungen eingehender erläutert. Sie kann alle hierin beschrieben Merkmale einzeln sowie in jedweder sinnvollen Kombination umfassen. In den Zeichnungen zeigen:
- Figur 1: einen Längsschnitt durch einen Katheter mit einem eingesetzten, erfindungsgemäßen Katheterstilett;
- Figur 2: einen vergrößerten Schnitt A-A aus Figur 1;
- Figur 3: eine Darstellung des Stilett-Katheter-Systems beim Ansetzen bzw. Abnehmen des Stiletts;
- Figuren 4 und 5: unterschiedliche Ausführungsformen eines gerollten Folien- Stiletts;
- Figuren 6 und 7: eine Hebelanordnung zur Handhabung eines gerollten Folien- Stiletts; und
- Figur 8: eine Abzieh- bzw. Öffnungsvorrichtung für einen erfindungsgemäßen Katheter.

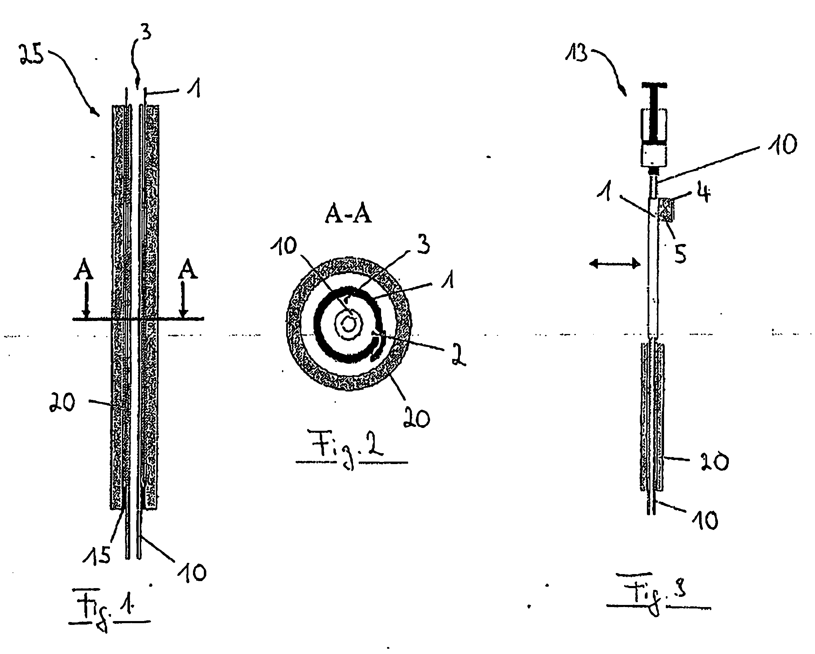

Die Figur 1 zeigt ein Katheterstilett 1 in einem erfindungsgemäßen Katheterstilett Kathetersystem. Das Kathetersystem weist die innere, flüssigkeitsführende Leitung 10 und die äußere Katheterhülle 20 auf, wobei das in Figur 1 dargestellte untere Ende der Leitung 10 das Austrittsende für das Medikament ist. Gestützt wird dieses Austrittsende 10 in der Hülle 20 durch ein buchsenartiges Element 15.

Wie in Figur'1 dargestellt ist, und wie auch der vergrößerten und explosionsartigen Ansicht der Figur 2 im Schnitt zu entnehmen ist, umgibt das erfindungsgemäße Stilett 1 die flüssigkeitsführende Leitung 10 des Katheters, und das Stilett selbst wird wiederum von der Katheterhülle (Hüllkatheterteil) 20 umgeben.

In dem Zustand, der in Figur 1 gezeigt ist, sorgt das Stilett 1, in dessen Lumen 3 die Leitung 10 liegt, für die Steifigkeit des Katheters beim Setzvorgang.

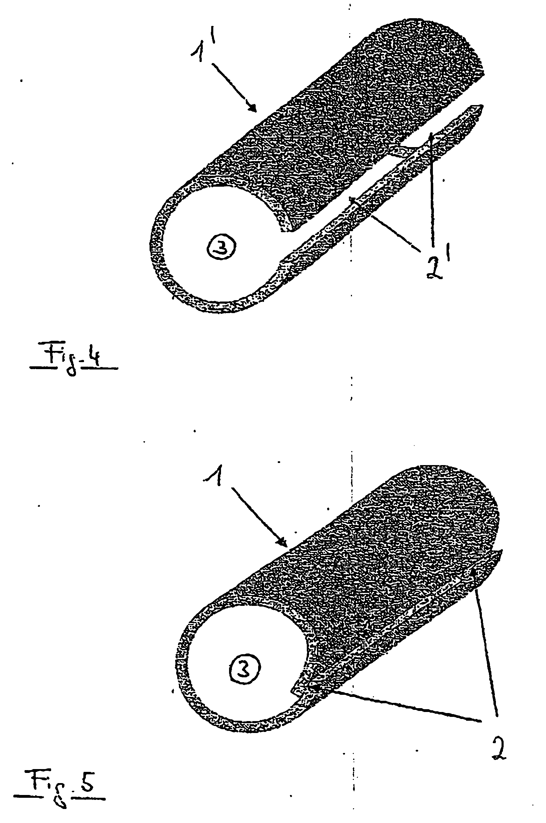

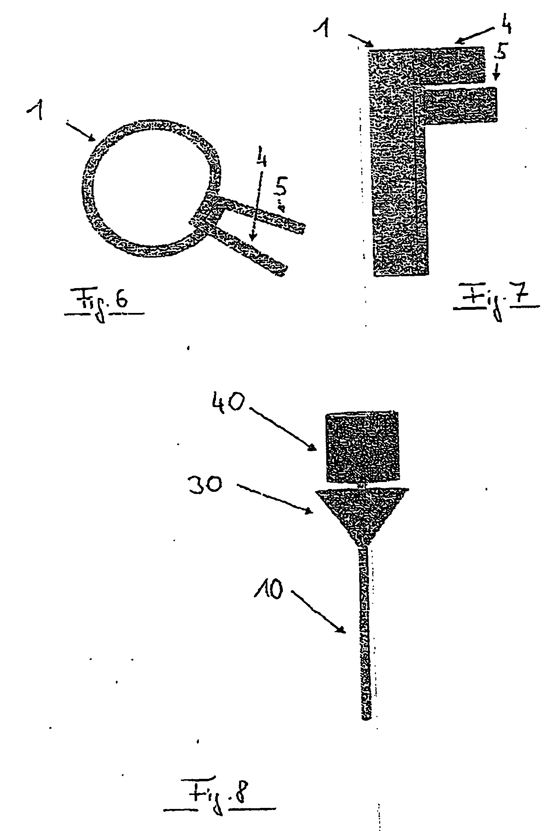

Anhand der Figur 3 kann erläutert werden, wie das Stilett 1 am Katheter angesetzt werden kann. Zunächst soll jedoch auf die Figuren 4 und 5 hingewiesen werden, welche zeigen, wie man ein solches Stilett 1' bzw. 1 aus einer Folie rollen kann, wobei der Längsspalt 2', 2 entstehen oder geschaffen werden kann. indem die Längskanten auseinander gehalten werden (Figur 4) bzw. überlappt werden (Figur 5). In den Figuren 6 und 7 wird dargestellt, wie am oberen Ende des Stiletts 1, das gemäß Figur 5 mit überlappenden Längskanten 2 ausgebildet ist, jeweils ein Hebel 4, 5 vorgesehen werden kann. Wenn diese Hebel 4, 5 zusammengedrückt werden, kann sich das Stilett 1 seitlich elastisch öffnen, um einen Zugang auszubilden und über die Leitung 10 gesetzt zu werden.

Geht man nun zur Figur 3 zurück, so wird ersichtlich, dass die flüssigkeitsführende bzw. medikamentenführende Leitung 10, die durch die Spritze 13 mit dem Medikament versorgt wird, so geprimed werden kann, dass sie vollständig mit dem Medikament befüllt ist. Dann kann an dem Teil der Leitung 10, der aus der Katheterhülle 20 nach oben herausragt, das erfindungsgemäße Stilett 1 angesetzt werden, indem die Hebel 4, 5 zunächst etwas zusammen gedrückt werden, um einen Zugang 2 zu schaffen. Wenn das Folienstilett sich dann elastisch wieder zusammenbiegt, wird es die Leitung 10 umschließen. Das Stilett 1 kann dann an der Leitung 10 hinab in die Katheterhülle 20 eingeführt werden; wenn notwendig wird es hierzu noch etwas mit Hilfe der Hebel 4, 5 geöffnet, um ein Gleiten an der Leitung 10 zu ermöglichen. Schließlich wird es zwischen der Leitung 10 und der Katheterhülle 20 in der Position sitzen, die in Figur 1 gezeigt ist, und der Katheter kann gesetzt werden.

Nach dem Setzen kann das Stilett 1 wieder in die Position der Figur 3 gebracht und seitlich bzw. quer (Pfeilrichtung) von der Leitung 10 abgenommen werden, ohne dass die Leitung 10 oder ihre Verbindung zur Spritze (oder auch Pumpe) 13 unterbrochen werden muss.

Um das Öffnen bzw. Abziehen solcher Stilette, aber auch von Stiletten, die aus gerollten Materialstreifen aufgebaut sind, zu erleichtem, kann gemäß Figur 8 an der flüssigkeitsführenden Leitung 10 vor dem Adapter 40 (z.B. Luer-Adapter zum Anschluss an die Spritze) ein Kegel- oder Trichterelement vorgesehen werden, welches in Figur 8 mit dem Bezugszeichen 30 gekennzeichnet worden ist. Dieses Kegelelement kann ein Stilett mit einem Längsspalt, das folienartig und rohrförmig ausgebildet ist, öffnen, wenn das Stilett gegen die Kegelspitze gedrückt wird, um so das Abnehmen des Stiletts von der Leitung 10 zu vereinfachen.

## Patentansprüche

1. Katheterstilett-Katheter-System mit einem Katheter (25) und einem Katheterstilett (1), wobei das Stilett (1) ein Aufnahmelumen (3) für zumindest eine innere flüssigkeitsführende Leitung (10) des Katheters (25) aufweist, **dadurch gekennzeichnet, dass** der Katheter (25) eine innere flüssigkeitsführende Leitung (10) und einen äußeren Hüllkatheterteil (20) aufweist, der die flüssigkeitsführende Leitung (10) umgibt, und das Stilett (1) zum Einsetzen zwischen die Leitung (10) und den Hüllkatheterteil (20) vorgesehen ist, so dass es die Leitung (10) umgreift und vom Hüllkatheterteil umgriffen wird.

2. System nach Anspruch 1, **dadurch gekennzeichnet, dass** an der flüssigkeitsführenden Leitung eine Abzieh- oder Öffnungsvorrichtung (30) angeordnet ist, insbesondere ein Kegel- oder Trichterelement.

3. System nach Anspruch 1 oder 2, **dadurch gekennzeichnet, dass** am Stilett (1) ein Zugang (2) für das Aufnahmelumen (3) vorhanden ist oder geschaffen werden kann, um zumindest die flüssigkeitsführende Leitung (10) des Katheters (25) in das Lumen (3) einzubringen oder aus dem Lumen (3) auszubringen.

4. System nach einem der Anspruche 1 bis 3, **dadurch gekennzeichnet, dass** das Stilett (1) einen im Wesentlichen zylinderförmigen oder rohrförmigen, länglichen Körper aufweist und das Aufnahmelumen (3) der innenraum des Körpers ist.

5. System nach einem der Ansprüche 3 oder 4, **dadurch gekennzeichnet, dass** der Zugang (2) derart gestaltet ist oder geschaffen werden kann, dass das Stilett (1) seitlich bzw. im wesentlichen quer zum Verlauf der Leitung (10) um diese herum angebracht oder von dieser abgezogen werden kann.

6. System nach einem der Ansprüche 3 bis 5, **dadurch gekennzeichnet, dass** der Zugang einen Längsschlitz (2) im Körper des Stiletts umfasst.

7. System nach einem der Ansprüche 3 bis 6, **dadurch gekennzeichnet, dass** der Zugang eine längs bzw. axial am Stilettkörper verlaufende Schwächungsstelle. insbesondere eine Perforation des Stilettmaterials, umfasst.

8. System nach einem der Ansprüche 3 bis 7, **dadurch gekennzeichnet, dass** der Zugang zwei überlappende Längskante (2') am Körper des Stiletts (1') umfasst.

9. System nach einem der Ansprüche 3 bis 8, **dadurch gekennzeichnet, dass** am Körper des Stiletts (1) eine Hebeleinrichtung (4, 5) angeordnet ist, mit welcher das Stilett gegriffen oder im Durchmesser verändert werden kann, insbesondere zum Öffnen oder Schließen eines Zugangs, der durch zwei überlappende oder aneinander anstoßende Stilettkörper-Längskanten gebildet wird.

10. System nach einem der Ansprüche 1 bis 9, **dadurch gekennzeichnet, dass** das Stilett aus einem Folienkörper gebildet ist. .

11. System nach einem der Ansprüche 1 bis 10, **dadurch gekennzeichnet, dass** das Stilett mehrere Material- oder Folienschichten umfasst, insbesondere eine unterschiedliche Anzahl von Schichten entlang der Stilettlänge.

12. System nach einem der Ansprüche 1 bis 11, **dadurch gekennzeichnet, dass** das Stilett durch mindestens einen im Winkel zusammengerollten Materialstreifen, insbesondere Folienstreifen gebildet wird.

13. System nach einem der Ansprüche 1 bis 12, **dadurch gekennzeichnet, dass** das Stilett teleskopartig aufgebaut ist.

14. System nach einem der Ansprüche 1 bis 13, **dadurch gekennzeichnet, dass** das Stilett eines oder mehrere der folgenden Materialien aufweist oder aus einem Material hergestellt ist, das eine oder mehrere der folgenden Materialeigenschaften aufweist
- ein Metall bzw. eine Metallfolie:
- ein Kunststoff bzw. eine Kunststoftfolie, insbesondere ein Polymer, speziell PE, PP oder PEEK;
- ein Kompositmaterial bzw. eine Kompositmaterialfolie:
- ein Memory-Metall oder eine Memory-Legierung;
- ein CT-kompatibles bzw. auf CT-Bildern sichtbares Material;
- ein MR-kompatibles bzw. auf MR-Bildern sichtbares Material;
- ein biokompatibles Material.

15. Verfahren zum Versteifen eines Katheters (25) vor dem Setzvorgang, bei dem ein Stilett (1), das ein Aufnahmelumen (3) aufweist, um zumindest eine flüssigkeitsführende Leitung (10) des Katheters (25) angeordnet wird, wobei ein System gemäß einem der Ansprüche 1 bis 14 verwendet wird.

## Claims

1. A catheter stylet catheter system comprising a catheter (25) and a catheter stylet (1), wherein the stylet (1) comprises an accommodating lumen (3) for at least one interior fluid-guiding line (10) of the catheter (25), **characterised in that** the catheter (25) comprises an interior fluid-guiding line (10) and an exterior covering catheter part (20) which surrounds the fluid-guiding line (10), and the stylet (1) is provided for insertion between the line (10) and the covering catheter part (20), such that it surrounds the line (10) and is surrounded by the covering catheter part.

2. The system according to claim 1, **characterised in that** a removing or opening device (30), in particular a cone element or funnel element, is arranged on the fluid-guiding line.

3. The system according to claim 1 or 2, **characterised in that** an access (2) for the accommodating lumen (3) is or can be provided on the stylet (1), in order to introduce at least the fluid-guiding line (10) of the catheter (25) into the lumen (3) or to remove it from the lumen (3).

4. The system according to any one of claims 1 to 3, **characterised in that** the stylet (1) comprises a substantially cylindrical or tubular, elongated body, and the accommodating lumen (3) is the interior space of the body.

5. The system according to any one of claims 3 or 4, **characterised in that** the access (2) is configured or can be provided such that the stylet (1) can be attached around the line (10) or removed from the line (10) laterally and/or substantially transverse to the profile of the line (10).

6. The system according to any one of claims 3 to 5, **characterised in that** the access comprises a longitudinal slit (2) in the body of the stylet.

7. The system according to any one of claims 3 to 6, **characterised in that** the access comprises a weak point running longitudinally and/or axially on the stylet body, in particular a perforation of the stylet material.

8. The system according to any one of claims 3 to 7, **characterised in that** the access comprises two overlapping longitudinal edges (2') on the body of the stylet (1').

9. The system according to any one of claims 3 to 8, **characterised in that** a lever device (4, 5) is arranged on the body of the stylet (1), using which the stylet can be gripped or altered in its diameter, in particular for opening or closing an access which is formed by two overlapping or mutually abutting stylet body longitudinal edges.

10. The system according to any one of claims 1 to 9, **characterised in that** the stylet is formed from a film body.

11. The system according to any one of claims 1 to 10, **characterised in that** the stylet comprises a plurality of material layers or film layers, in particular a different number of layers along the length of the stylet.

12. The system according to any one of claims 1 to 11, **characterised in that** the stylet is formed by at least one material strip, in particular film strip, which is rolled together at an angle.

13. The system according to any one of claims 1 to 12, **characterised in that** the stylet is formed telescopically.

14. The system according to any one of claims 1 to 13, **characterised in that** the stylet comprises one or more of the following materials or is made from a material which exhibits one or more of the following material properties:
- a metal or metal film;
- a plastic or plastic film, in particular a polymer, especially PE, PP or PEEK;
- a composite material or composite material film;
- a memory metal or memory alloy;
- a material which is CT-compatible and/or visible in CT images;
- a material which is MR-compatible and/or visible in MR images;
- a biocompatible material.

15. A method for stiffening a catheter (25) before the placing process, wherein a stylet (1) which comprises an accommodating lumen (3) is arranged around at least one fluid-guiding line (10) of the catheter (25), wherein a system in accordance with any one of claims 1 to 14 is used.

## Revendications

1. Stylet de cathéter destiné à rigidifier un cathéter (25) pendant le processus de mise en place, **caractérisé en ce que** le stylet (1) comporte un conduit de réception (3) pour au moins une ligne de guidage de fluide (10) du cathéter (25).

2. Stylet de cathéter selon la revendication 1, **caractérisé en ce qu'**un accès (2) pour la lumière de réception (3) existe ou peut être créé sur le stylet (1) afin d'introduire au moins la ligne de guidage de fluide (10) du cathéter (25) dans le conduit (3) ou de l'extraire du conduit (3).

3. Stylet de cathéter selon la revendication 1 ou 2, **caractérisé en ce que** le stylet (1) comporte un corps allongé, sensiblement cylindrique ou tubulaire, et le conduit de réception (3) est l'espace intérieur du corps.

4. Stylet de cathéter selon l'une quelconque des revendications 2 ou 3, **caractérisé en ce que** l'accès (2) est configuré ou peut être créé de telle sorte que le stylet (1) peut être fixé autour de la ligne (10) ou peut être retiré de celle-ci de manière latérale ou sensiblement transversale au profil de la ligne (10).

5. Stylet de cathéter selon l'une quelconque des revendications 2 à 4, **caractérisé en ce que** l'accès comporte une fente longitudinale (2) dans le corps du stylet.

6. Stylet de cathéter selon l'une quelconque des revendications 2 à 5, **caractérisé en ce que** l'accès comporte un point d'affaiblissement s'étendant longitudinalement ou axialement sur le corps de stylet, en particulier une perforation du matériau de stylet.

7. Stylet de cathéter selon l'une quelconque des revendications 2 à 6, **caractérisé en ce que** l'accès comporte deux bords longitudinaux chevauchants (2') sur le corps du stylet (1').

8. Stylet de cathéter selon l'une quelconque des revendications 2 à 7, **caractérisé en ce qu'**un dispositif à levier (4, 5) est agencé sur le corps du stylet (1), au moyen duquel le stylet peut être saisi ou changé en diamètre, en particulier pour ouvrir ou fermer un accès qui est formé par deux bords longitudinaux de corps de stylet qui se chevauchent ou qui sont adjacents l'un à l'autre.

9. Stylet de cathéter selon l'une quelconque des revendications 1 à 8, **caractérisé en ce que** le stylet est formé à partir d'un corps de film.

10. Stylet de cathéter selon l'une quelconque des revendications 1 à 9, **caractérisé en ce que** le stylet inclut plusieurs couches de matériau ou de film, en particulier un nombre différent de couches le long de la longueur du stylet.

11. Stylet de cathéter selon l'une quelconque des revendications 1 à 10, **caractérisé en ce que** le stylet est formé par au moins une bande de matériau enroulée sur elle-même selon un certain angle, en particulier une bande de film.

12. Stylet de cathéter selon l'une quelconque des revendications 1 à 11, **caractérisé ce que** le stylet est formé de manière télescopique.

13. Stylet de cathéter selon l'une quelconque des revendications 1 à 12, **caractérisé en ce que** le stylet comporte un ou plusieurs des matériaux suivants, ou est fabriqué à partir d'un matériau qui présente une ou plusieurs des propriétés de matériau suivantes :
- un métal ou un film de métal,
- une matière plastique ou un film de matière plastique, en particulier un polymère, notamment PE, PP ou PEEK,
- un matériau composite ou un film de matériau composite,
- un métal à mémoire de forme ou un alliage à mémoire de forme.

14. Système selon l'une quelconque des revendications 1 à 13, **caractérisé en ce que** le stylet comporte un ou plusieurs des matériaux suivants, ou est fabriqué à partir d'un matériau qui présente une ou plusieurs des propriétés de matériau suivantes :
- un métal ou un film de métal,
- une matière plastique ou un film de matière plastique, en particulier un polymère, notamment PE, PP ou PEEK,
- un matériau composite ou un film de matériau composite,
- un métal à mémoire de forme ou un alliage à mémoire de forme,
- un matériau compatible avec la CT ou visible sur des images CT,
- un matériau compatible avec la MR ou visible sur des images MR,
- un matériau biocompatible.

15. Procédé pour rigidifier un cathéter (25) avant le processus de mise en place, dans lequel un stylet (1) comportant un conduit de réception (3) est agencé autour d'au moins une ligne de guidage de fluide (10) du cathéter (25), dans lequel ou utilise un système selon l'une quelconque des revendications 1 à 14.
